# EUROPÄISCHE PATENTSCHRIFT

(11) **EP 1 304 362 B1**
(45) Veröffentlichungstag und Bekanntmachung des Hinweises auf die Patenterteilung: **10.03.2004**
(21) Anmeldenummer: 02020731.2
(22) Anmeldetag: 14.09.2002
(51) Int. Cl.: C09C 1/56, C09C 3/08, C08K 9/04, C07D 249/18, D01F 11/14

(54) **Kohlenstoffhaltiges Material**
Carbonaceous material
Matériau carboné

(30) Priorität: 09.10.2001 DE 10149805
(43) Veröffentlichungstag der Anmeldung: 23.04.2003
(73) Patentinhaber: Degussa AG, 40474 Düsseldorf (DE)
(72) Erfinder: Bergemann, Klaus, Dr., 50170 Kerpen-Sindorf (DE); Fanghänel, Egon, Prof. Dr., 06114 Halle/S. (DE); Knackfuss, Bernd, Dr., 04229 Leipzig (DE); Lüthge, Thomas, Dr., 63454 Hanau (DE)

(56) Entgegenhaltungen:
- EP-A- 1 134 261
- EP-A- 1 136 526
- WO-A-96/18690

## Beschreibung

Die Erfindung betrifft ein kohlenstoffhaltiges Material, ein Verfahren zu seiner Herstellung sowie seine Verwendung.

Aus EP 0569503 ist ein Verfahren zur Oberflächenmodifizierung von kohlenstoffhaltigem Material mit aromatischen Gruppen durch elektrochemische Reduktion eines Diazoniumsalzes bekannt.

Weiterhin ist bekannt, Ruß mit organischen Gruppen zu versehen, indem man die organischen Gruppen über eine Diazotierung mit dem kohlenstoffhaltigen Material verknüpft (WO 96/18690) oder die organischen Gruppen mittels Umsetzungen mit Radikalbildnern (Ohkita K., Tsubokawa N., Saitoh E., Carbon 16 (1978) 41), DE 10012784.3) oder über Cycloadditionen (DE 10012783.5, JP11315220 A) an den Ruß bindet.

Die bekannten Verfahren haben folgende Nachteile:
◆ Die, neben dem giftigen und brandfördernden Natriumnitrit, ebenfalls zur Diazotierung verwendbaren nichtionischen organischen Nitrite sind giftig und leicht brennbar. Reste der Nitrite (Gegenionen, Alkylreste) verbleiben ungebunden als Verunreinigung im Ruß.
◆ Zur Durchführung der Diazotierung ist der Einsatz von Nitrit im sauren Medium erforderlich. Dabei können sich giftige Stickstoffoxide bilden.
◆ Radikalbildner sind thermisch beziehungsweise photochemisch labil, explosionsgefährlich und können zu schwer beherrschbaren Kettenreaktionen führen.
◆ Die Synthese und Reinigung der entsprechenden Vorstufen der Radikalbildner laufen zum Teil über giftige beziehungsweise geruchsbelästigende Stoffe.
◆ Die bei Cyclisierungsreaktionen mit Stickstoffheterocyclen ablaufende Stickstoffextrusion kann zu plötzlichen, explosionsartigen Volumenausdehnungen beziehungsweise Druckanstiegen führen, die die Reaktionsführung wesentlich erschweren.

Aufgabe der Erfindung ist es, ein kohlenstoffhaltiges Material mit organischen Gruppen zur Verfügung zu stellen, wobei
- die Modifizierung des kohlenstoffhaltigen Materials so variabel ist, daß die Gruppen direkt über der Oberfläche des kohlenstoffhaltigen Materials angeordnet und/oder auch sehr weit von der Oberfläche des kohlenstoffhaltigen Materials entfernt sein können,
- die Modifizierung des kohlenstoffhaltigen Materials ohne vorgelagerte Reaktionen, wie Aktivierung durch Starter, abläuft,
- die Umsetzungen mit den erfindungsgemäß verwendeten Modifizierungsmitteln thermisch ablaufen und keine Katalysatoren (zum Beispiel Lewissäuren) benötigt werden,
- auf Grund der chemischen Eigenschaften der erfindungsgemäß verwendeten Modifizierungsmittel keine störenden Nebenreaktionen oder schwer zu kontrollierende Kettenreaktionen ablaufen können,
- das resultierende kohlenstoffhaltige Material nicht durch Säuren, Salze und ähnliches verunreinigt ist, so dass keine Reinigung des kohlenstoffhaltigen Materials erforderlich ist,
- das kohlenstoffhaltige Material nicht mit hohem Energieaufwand getrocknet werden muß,
- keine giftigen Abgase bei der Modifizierung entstehen und
- keine oder nur geringe Mengen leicht entfernbarer Lösemittel erforderlich sind.

Gegenstand der Erfindung ist ein kohlenstoffhaltiges Material mit organischen Gruppen, welches dadurch gekennzeichnet ist, daß dieses erhältlich ist durch die Umsetzung von kohlenstoffhaltigem Material mit organischen Verbindungen der allgemeinen Formel 1, 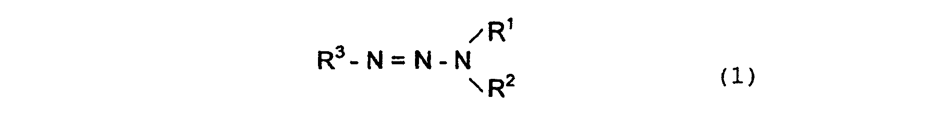 wobei R¹, R² und R³ gleich oder verschieden sind und aus H, Alkyl- oder Arylgruppen mit Akzeptor- beziehungsweise Donatorsubstituenten und/oder hydrophilen beziehungsweise hydrophoben Gruppen bestehen oder
R¹ und R³ mit den drei N-Atomen ein cyclisches System bilden und
R² aus H, Alkyl- oder Arylgruppen mit Akzeptor- beziehungsweise Donatorsubstituenten und/oder hydrophilen beziehungsweise hydrophoben Gruppen besteht.

Das cyclische System kann Stickstoff, Kohlenstoff oder weitere Heteroatome, beispielsweise Schwefel oder Sauerstoff, enthalten und beispielsweise ein Triazol, Tetrazol oder Pentazol sein.

Als Triazolverbindungen können 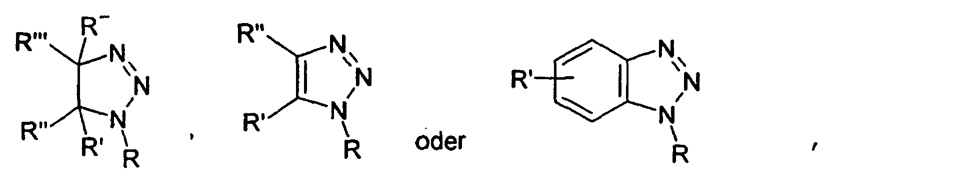 wobei R, R', R", R''', R'''' gleich oder verschieden sind und aus H, Alkyl- oder Arylgruppen mit Akzeptor- beziehungsweise Donatorsubstituenten oder Teile von cyclischen Systemen mit Akzeptor- beziehungsweise Donatorsubstituenten und/oder hydrophilen beziehungsweise hydrophoben Gruppen bestehen, eingesetzt werden.

Insbesondere können als Triazolverbindungen 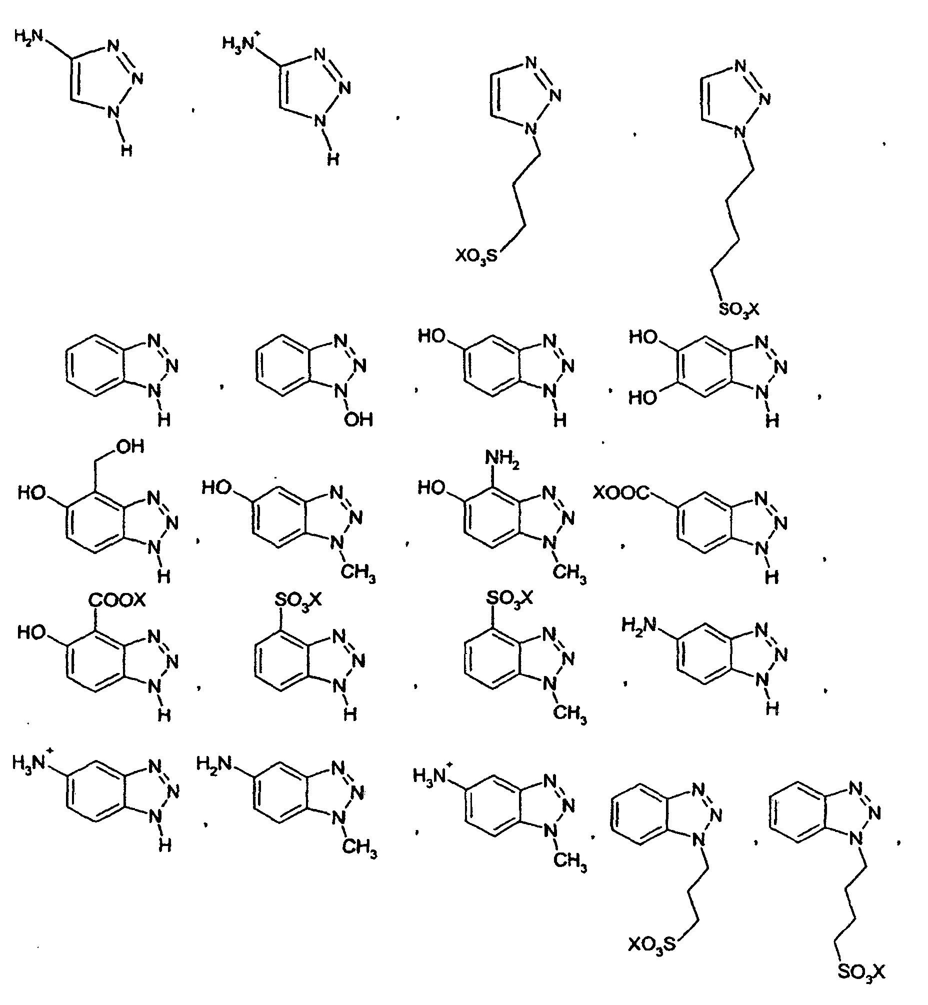 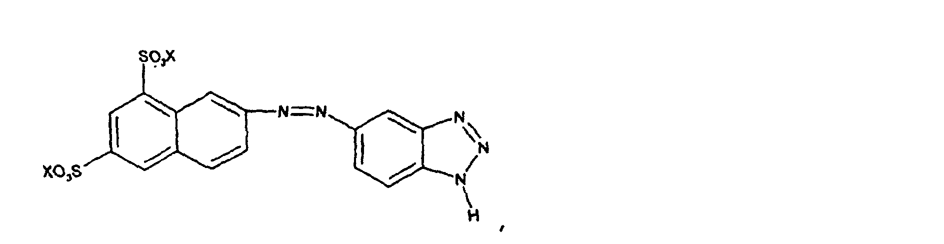 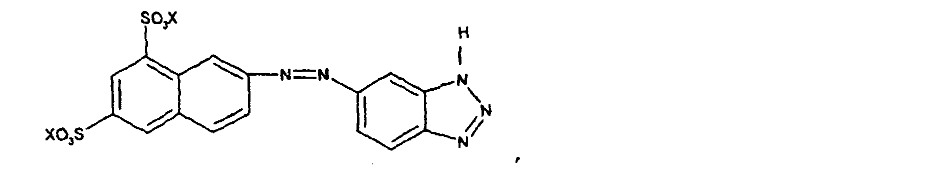 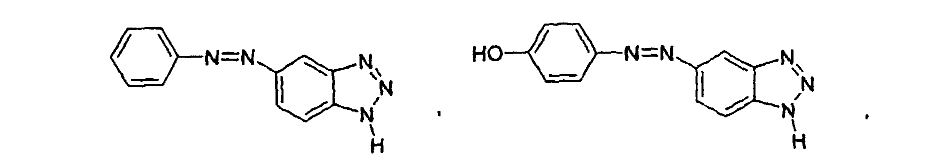 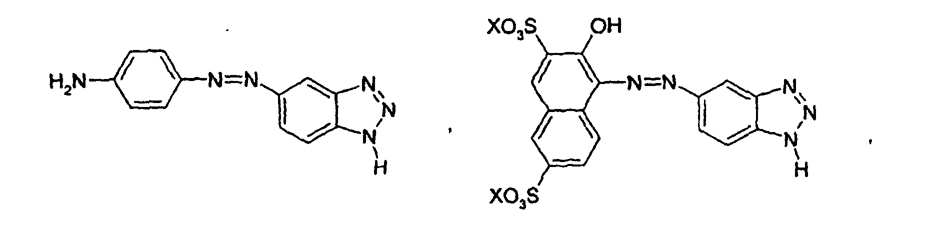 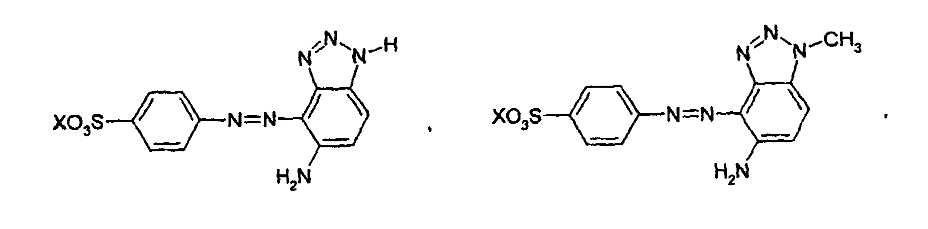 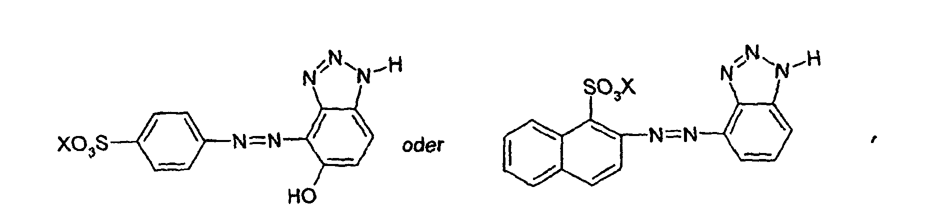 mit X=H, Metallⁿ⁺ (n = 1-3), N⁺R₄, eingesetzt werden.

Akzeptorsubstituenten können -COOR⁴, -CO-R⁴, -CN, -SO₂R⁴ oder -SO₂OR⁴ mit R⁴ = H, Alkyl, Aryl oder funktionalisiertes Alkyl oder Aryl, wie zum Beispiel ω-Carboxyalkyl, HSO₃-CₓH_{y}-, H₂N-CₓH_{y}- oder H₂N-SO₂-CₓH_{y}- (x,y=1-5), sein.

Donatorsubstituenten können Alkyl-, Arylgruppen, OR⁵ oder N(R⁵)₂ mit R⁵ = H, Alkyl, Aryl, oder funktionalisiertes Alkyl oder Aryl sein.

Die organischen Gruppen R¹, R² und R³ können:
- eine aliphatische Gruppe umfassen, eine cyclische organische Gruppe oder eine organische Verbindung mit einem aliphatischen und einem cyclischen Teil sein,
- substituiert oder unsubstituiert, verzweigt oder unverzweigt sein,
- eine aliphatische Gruppe umfassen, beispielsweise Reste aus Alkanen, Alkenen, Alkoholen, Ethern, Aldehyden, Ketonen, Carbonsäuren, Kohlenwasserstoffen, Sulfonsäuren, Trialkylammonium-, Trialkylphosphonium-, Dialkylsulfonium-,
- eine cyclische Verbindung sein, beispielsweise alicyclische Kohlenwasserstoffe, wie zum Beispiel Cycloalkyle oder Cycloalkenyle, heterocyclische Verbindungen, wie zum Beispiel Pyrrolidinyl-, Pyrrolinyl-, Piperidinyl- oder Morpholinyl-, Arylgruppen, wie zum Beispiel Phenyl-, Naphthyl- oder Anthracenyl- und Heteroarylgruppen, wie zum Beispiel Imidazolyl-, Pyrazolyl-, Pyridinyl-, Thienyl-, Thiazolyl-, Furyl- oder Indolyl-,
- durch weitere funktionelle Gruppen substituiert sein,
- eine chromophore Gruppe oder ein Farbstoff sein,
- geeignete reaktive Verbindungen, wie zum Beispiel Triarylammonium-, Triarylphosphonium-, Diarylsulfonium- und Aryliodonium- sein.

Verbindungen der allgemeinen Formel 1 mit R¹, R² oder R³ = H können nachträglich durch Alkylierungs- oder Arylierungsreaktionen funktionalisiert werden. Die primär aufgebrachten Gruppen können durch weitere Folgereaktionen darüber hinaus modifiziert werden.

Die Substituenten der organischen Verbindungen der allgemeinen Formel 1 können dabei auf die potentiellen Anwendungsgebiete maßgeschneidert werden, da das Reaktionsprinzip beispielsweise die Einführung von hydrophilen, wie auch von lipophilen Substituenten gestattet. Die Substituenten können auch ionisch, polymer oder für weitere Reaktionen reaktiv sein. Über die Substituenten können unterschiedliche, anwendungstechnisch hoch interessante Eigenschaften des kohlenstoffhaltigen Materials gezielt verändert werden. So kann beispielsweise die Hydrophilie des kohlenstoffhaltigen Materials so weit gesteigert werden, daß das kohlenstoffhaltige Material ohne Verwendung eines Netzmittels in wässrigen Medien stabile Dispersionen bildet.

Als kohlenstoffhaltiges Material kann Ruß, Graphitpulver, Graphitfasern, Kohlenstofffasern, Kohlenstofffibrillen, Kohlenstoffnanoröhren (Carbon Nanotubes), Kohlenstoffgewebe, glasartige Kohlenstoffprodukte und Aktivkohle verwendet werden.

Als Ruß kann jeder bekannte Ruß, wie zum Beispiel Furnaceruß, Gasruß, Channelruß, Flammruß, Thermalruß, Acetylenruß, Plasmaruß, Inversionsruße, bekannt aus DE 195 21 565, Si-haltige Ruße, bekannt aus WO 98/45361 oder DE 19613796, oder metallhaltige Ruße, bekannt aus WO 98/42778, Lichtbogenruß und Ruße, die Nebenprodukte chemischer Produktionsprozesse sind, verwendet werden. Der Ruß kann durch vorgelagerte Reaktionen aktiviert werden. Es können Ruße, die als Verstärkerfüllstoff in Kautschukmischungen verwendet werden, eingesetzt werden. Es können Farbruße eingesetzt werden. Weitere Ruße können sein: Leitfähigkeitsruß, Ruß zur UV-Stabilisierung, Ruß als Füllstoff in anderen Systemen als Kautschuk, wie zum Beispiel in Bitumen, Kunststoff, Ruß als Reduktionsmittel in der Metallurgie.

Ein weiterer Gegenstand der Erfindung ist ein Verfahren zur Herstellung des erfindungsgemäßen kohlenstoffhaltigen Materials, welches dadurch gekennzeichnet ist, daß man kohlenstoffhaltiges Material mit organischen Verbindungen der allgemeinen Formel 1 umsetzt.

Die organische Verbindung kann auf das kohlenstoffhaltige Material aufgebracht werden durch Einmischen oder Aufsprühen. Die organische Verbindung kann als Pulver, Schmelze oder Lösung aufgebracht werden. Besonders vorteilhaft kann die Aufbringung der organischen Verbindung während der Herstellung des kohlenstoffhaltigen Materials sein, wobei die Zugabe der organischen Verbindung vorzugsweise an einem Ort erfolgt, der die notwendige Temperatur aufweist. Die Reaktion zur Modifizierung des kohlenstoffhaltigen Materials kann vorzugsweise lösungsmittelfrei, aber auch in einem Lösungsmittel, vorzugsweise leicht flüchtigen organischen Lösungsmittel, durchgeführt werden. Die Reaktion zur Modifizierung des kohlenstoffhaltigen Materials kann bei Temperaturen von -80° bis 250°C, vorzugsweise von 80° bis 180°C, durchgeführt werden. Der Energieeintrag kann mittels mechanischer Energie, Schwingungsenergie, beispielsweise Ultraschall, oder Strahlungsenergie, beispielsweise Mikrowellenstrahlung, Wärmestrahlung, Lichtstrahlung, Röntgenund Elektronenstrahlung, erfolgen.

Die erfindungsgemäßen kohlenstoffhaltigen Materialien mit organischen Gruppen können beispielsweise als Füllstoff, Verstärkerfüllstoff, UV-Stabilisator, Leitfähigkeitsruß oder Pigment in Kautschuk, Kunststoff, Druckfarben, Tinten, Inkjet-Tinten, Lacken, Toner und Farben, Bitumen, Beton und anderen Baustoffen oder Papier eingesetzt werden. Ferner können die erfindungsgemäßen kohlenstoffhaltigen Materialien als Reduktionsmittel in der Metallurgie verwendet werden.

Ein weiterer Gegenstand der Erfindung ist eine organische Verbindung zur Herstellung des erfindungsgemäßen kohlenstoffhaltigen Materials mit organischen Gruppen, welche dadurch gekennzeichnet ist, dass diese der allgemeinen Formel 2 oder 3 entspricht, 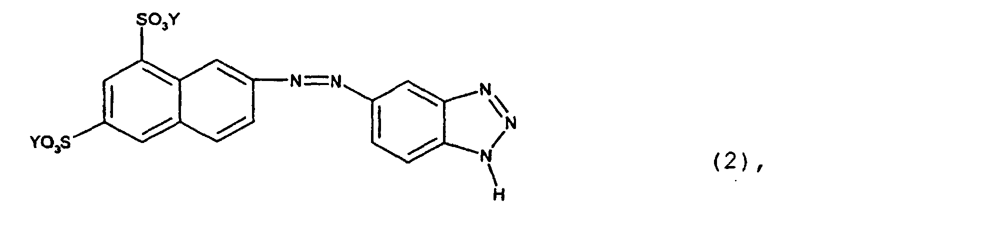 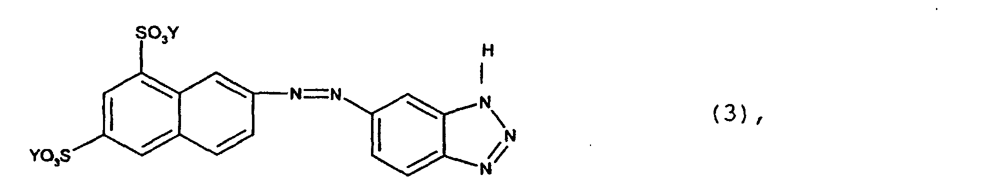 wobei Y H, Metall^{u+} (u=1-3) oder N⁺R₄ bedeutet.

Ein weiterer Gegenstand der Erfindung ist eine Dispersion, welche dadurch gekennzeichnet ist, dass sie erfindungsgemäßes kohlenstoffhaltiges Material mit organischen Gruppen enthält.

Die erfindungsgemäße Dispersion kann in Druckfarben, Tinten, Lacken, Toner und Farben verwendet werden.

Die erfindungsgemäßen kohlenstoffhaltigen Materialien weisen den Vorteil auf, dass
- polar modifizierte kohlenstoffhaltige Materialien (zum Beispiel mit -SO₃⁻-Substituenten) besser in polaren Systemen, vorrangig Wasser, dispergierbar sind,
- unpolar modifizierte kohlenstoffhaltige Materialien (zum Beispiel mit Alkylgruppen) besser in unpolaren Systemen, wie zum Beispiel Ölen, dispergierbar sind,
- geeignet modifiziertes kohlenstoffhaltige Materialien mit polaren oder sterisch sperrigen Gruppen in den Systemen elektrostatisch, beziehungsweise sterisch stabilisiert werden und zur Stabilisierung keine weiteren Hilfsstoffe wie zum Beispiel Netzmittel notwendig sind,
- nach dem erfindungsgemäßen Verfahren modifizierte kohlenstoffhaltige Materialien besser in Dispersionen stabilisiert sind und so bessere koloristische Eigenschaften, wie Farbtiefe und Blaustichigkeit, aufweisen,
- kohlenstoffhaltige Materialien mit gebundenen Farbstoffen veränderte Farbtöne aufweisen,
- kohlenstoffhaltige Materialien mit weiterhin reaktiven Substituenten zur Kopplung und Vernetzung in Systemen (zum Beispiel Kautschuk), genutzt werden können,
- reaktiv modifizierte kohlenstoffhaltige Materialien eine Anbindung des kohlenstoffhaltigen Materialien an das Polymer ermöglichen,
- kohlenstoffhaltige Materialien dabei arm an Nebenprodukten, Salzen, Säuren und Feuchtigkeit hergestellt werden können.

### Beispiele

Als Ruße werden in den Beispielen FW1 und Printex 35 eingesetzt; die Modifizierungen sind aber nicht auf diese Rußtypen begrenzt. Die genannten Ruße sind Produkte der Firma Degussa AG. Elementaranalysen werden mit einem Leco CHNS-932 bestimmt. Zur Aufnahme der ¹H-NMR-Spektren (300 MHz) wird ein Varian Gemini 300 (δ-Skala, interner Standart TMS) verwendet. Die dynamische und statische Oberflächenspannung werden mit dem Blasentensiometer BP2 der Fa. Krüss, die Viskosität mit einem Physica US 200 (Doppelspaltmesssystem) und der pH-Wert mit dem pH-Meter CG 837 gemessen.

### Beispiel 1:

Synthese und Charakterisierung von 1H-Benzotriazol-5-azo-2'naphthyl-6',8'-disulfonsäure Dikaliumsalz. 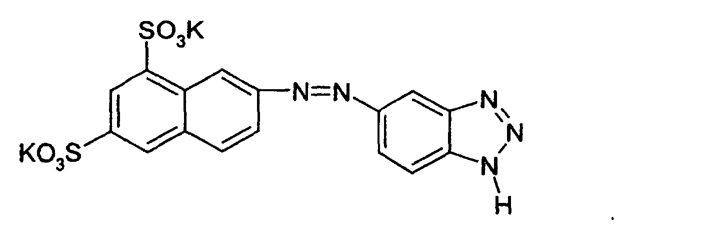 3 g 2-Naphthylamin-6,8-disulfonsäure Monokaliumsalz x H₂O und 1,5 g NaBF₄ werden in 30 ml Wasser eingerührt und durch Zugabe von wenig KOH gelöst. Anschließend wird das Amin mit konz. HCl feindispers ausgefällt, die Dispersion auf 0 °C abgekühlt und eine Lösung von 0,7 g NaNO₂ in 5 ml Wasser langsam zugetropft. Nach Ende des Zutropfens wird 20 Minuten bei 0 °C nachgerührt und dann abgesaugt. Der Filterrückstand wird portionsweise zu einer auf 5 - 10 °C abgekühlten Lösung von 1,2 g 1H-Benzotriazol und 1 g KOH in 30 ml Wasser gegeben. Die Lösung wird 30 Minuten gerührt und das Produkt danach mit 400 ml Aceton ausgefällt.
Molekulargewicht: 509 g/mol
Ausbeute: 95 %
Elementaranalyse [%]:
berechnet: C - 37,71; H - 1,78; N - 13,74; S - 12.58
gefunden: C - 38,05; H - 1,53; N - 13,89; S - 12.27
¹H-NMR δ [ppm] (J [Hz]); DMSO_{d6}:
6,15 (s 1H); 6,93 (d 1H, J = 9,9); 7,03 (d 1H, J = 10,2); 7,73 (s 1H); 7,82 (d 1H, J = 9,0); 8,03 (d 1H, J = 9,0); 8,16 (s 1H); 8,34 (s 1H) ; 9,45 (s 1H).

### Modifizierung von Ruß in fester Phase mit 1H-Benzotriazol-5-azo-2'-naphthyl-6',8'-disulfonsäure Dikaliumsalz

2 g 1H-Benzotriazol-5-azo-2'-naphthyl-6',8'-disulfonsäure Dikaliumsalz werden in 150 ml Wasser gelöst, zu der Lösung 10 g Ruß FW 1 gegeben und danach das Lösungsmittel im Vakuum abdestilliert. Im Anschluß daran erhitzt man das Gemisch 10 Stunden auf 180 °C. Der modifizierte Ruß wird mit 300 ml Wasser gewaschen und danach acht Stunden bei 100 °C getrocknet.

### Beispiel 2:

### Modifizierung von Ruß in Wasser mit 1H-Benzotriazol-5-azo-2'naphthyl-6',8'-disulfonsäure Dikaliumsalz .

2 g 1H-Benzotriazol-5-azo-2'-naphthyl-6',8'-disulfonsäure Dikaliumsalz werden in 200 ml Wasser gelöst. Zu der Lösung gibt man 10 g Ruß FW 1. Anschließend wird zwölf Stunden unter Rückfluß gekocht. Danach wird der Ruß abgesaugt und der Filterrückstand mit 400 ml Wasser gewaschen. Der modifizierte Ruß wird acht Stunden bei 100 °C getrocknet.

### Beispiel 3:

Modifizierung von Ruß in fester Phase mit ω-(1-Benzotriazolyl)-butansulfonsäure Natriumsalz

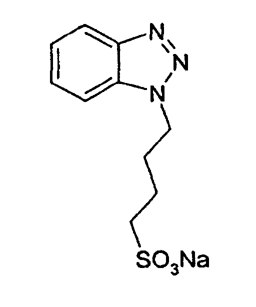

2 g ω-(1-Benzotriazolyl)-butansulfonsäure Natriumsalz werden in 250 ml Wasser gelöst, zu der Lösung 10 g Ruß Printex 35 gegeben und danach das Lösungsmittel im Vakuum abdestilliert. Im Anschluß daran erhitzt man das Gemisch 6 Stunden auf 180 °C. Der modifizierte Ruß wird mit 250 ml Wasser gewaschen und danach acht Stunden bei 100 °C getrocknet.

### Beispiel 4:

### Modifizierung von Ruß in Wasser mit ω-(1-Benzotriazolyl)-butansulfonsäure Natriumsalz

2 g ω-(1-Benzotriazolyl)-butansulfonsäure Natriumsalz werden in 150 ml Wasser suspendiert. Zu der Suspension gibt man 10 g Ruß Printex 35. Anschließend wird 8 Stunden unter Rückfluß gekocht. Danach wird der Ruß abgesaugt und der Filterrückstand mit 350 ml Wasser gewaschen. Der modifizierte Ruß wird acht Stunden bei 100 °C getrocknet.

### Beispiel 5:

### Dispersion von modifiziertem Ruß in Wasser

15 g funktionalisierter Ruß gemäß Beispiel 1 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.

Dynamische Oberflächenspannung bei 15 ms: 63 mN/m
Statische Oberflächenspannung bei 3000 ms: 60 Nm/m
pH-Wert: 7,5
Viskosität: 2,5 mPas

### Beispiel 6:

### Dispersion von modifiziertem Ruß in Wasser

15 g funktionalisierter Ruß gemäß Beispiel 2 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.

Dynamische Oberflächenspannung bei 15 ms: 59 mN/m
Statische Oberflächenspannung bei 3000 ms: 55 Nm/m
pH-Wert: 7,3
Viskosität: 3,0 mPas

### Beispiel 7:

### Dispersion von modifiziertem Ruß in Wasser

15 g funktionalisierter Ruß gemäß Beispiel 3 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.

Dynamische Oberflächenspannung bei 15 ms: 65 mN/m
Statische Oberflächenspannung bei 3000 ms: 61 Nm/m
pH-Wert: 7,8
Viskosität: 2,5 mPas

### Beispiel 8:

### Dispersion von modifiziertem Ruß in Wasser

15 g funktionalisierter Ruß gemäß Beispiel 4 werden mit 85 ml Wasser verrührt und anschließend 30 Minuten bei 5000 U/min mittels Ultra Turrax dispergiert. Die erhaltene Dispersion ist ohne weitere Zugabe von Netzmittel stabil.

Dynamische Oberflächenspannung bei 15 ms: 62 mN/m Statische Oberflächenspannung bei 3000 ms: 59 Nm/m
pH-Wert: 1,7
Viskosität: 2,5 mPas

## Patentansprüche

1. Kohlenstoffhaltiges Material mit organischen Gruppen, **dadurch gekennzeichnet, daß** dieses erhältlich ist durch die Umsetzung von kohlenstoffhaltigem Material mit organischen Verbindungen der allgemeinen Formel 1, 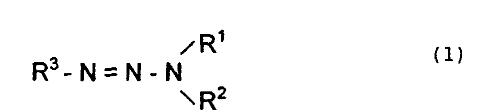 wobei R¹, R² und R³ gleich oder verschieden sind und aus H, Alkyl- oder Arylgruppen mit Akzeptor- beziehungsweise Donatorsubstituenten und/oder hydrophilen beziehungsweise hydrophoben Gruppen bestehen oder R¹ und R³ mit den drei N-Atomen ein cyclisches System bilden und R² aus H, Alkyl- oder Arylgruppen mit Akzeptor- beziehungsweise Donatorsubstituenten und/oder hydrophilen beziehungsweise hydrophoben Gruppen besteht.

2. Kohlenstoffhaltiges Material mit organischen Gruppen nach Anspruch 1, **dadurch gekennzeichnet, daß** die Akzeptorsubstituenten -COOR⁴, -CO-R⁴, -CN, -SO₂R⁴, -SO₂OR⁴ mit R⁴ = H, Alkyl, Aryl oder funktionalisiertes Alkyl oder Aryl sind.

3. Kohlenstoffhaltiges Material mit organischen Gruppen nach Anspruch 1, **dadurch gekennzeichnet, dass** die Donatorsubstituenten Alkyl-, Arylgruppen, OR⁵ oder N(R⁵)₂ mit R⁵ = H, Alkyl, Aryl, oder funktionalisiertes Alkyl oder Aryl sind.

4. Kohlenstoffhaltiges Material mit organischen Gruppen nach Anspruch 1, **dadurch gekennzeichnet, dass** das kohlenstoffhaltige Material Ruß ist.

5. Verfahren zur Herstellung des kohlenstoffhaltigen Materials nach Anspruch 1, **dadurch gekennzeichnet, daß** man kohlenstoffhaltiges Material mit organischen Verbindungen der allgemeinen Formel 1 umsetzt.

6. Verwendung des kohlenstoffhaltigen Materials nach Anspruch 1 als Füllstoff, Verstärkerfüllstoff, UV-Stabilisator, Leitfähigkeitsruß oder Pigment in Kautschuk, Kunststoff, Druckfarben, Tinten, Inkjet-Tinten, Lacken, Toner und Farben, Bitumen, Beton und anderen Baustoffen oder Papier.

7. Organische Verbindung zur Herstellung des kohlenstoffhaltigen Materials mit organischen Gruppen nach Anspruch 1, **dadurch gekennzeichnet, dass** diese der allgemeinen Formel 2 oder 3 entspricht, 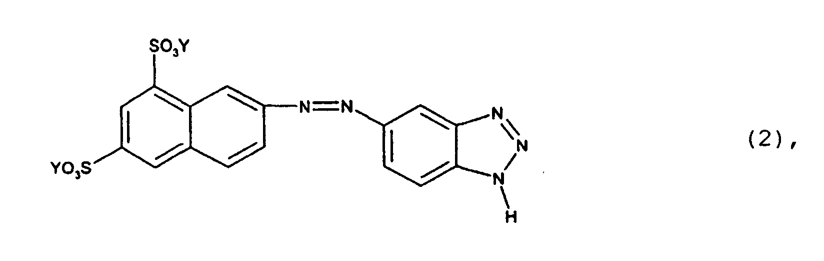 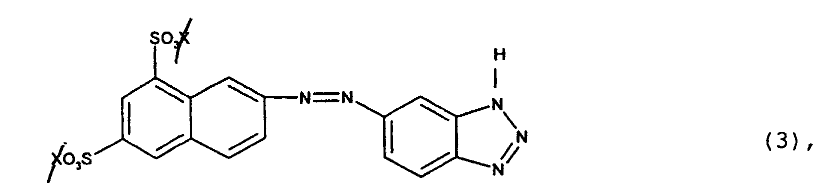 wobei Y = H, Metall^{u}* (u = 1-3) oder N⁺R₄ bedeutet.

8. Dispersion, **dadurch gekennzeichnet, dass** sie kohlenstoffhaltiges Material mit organischen Gruppen nach Anspruch 1 enthält.

9. Verwendung der Dispersion nach Anspruch 8 in Druckfarben, Tinten, Lacken, Toner und Farben.

## Claims

1. Carbon-containing material with organic groups, **characterized in that** this is obtainable by reaction of the carbon-containing material with organic compounds of the general formula 1, 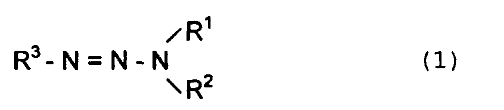 wherein R¹, R² and R³ are identical or different and comprise H or alkyl or aryl groups with acceptor or donor substituents and/or hydrophilic or hydrophobic groups or R¹ and R³ form a cyclic system with the three N atoms and R² comprises H or alkyl or aryl groups with acceptor or donor substituents and/or hydrophilic or hydrophobic groups.

2. Carbon-containing material with organic groups according to claim 1, **characterized in that** the acceptor substituents are -COOR⁴, -CO-R⁴, -CN, -SO₂R⁴, -SO₂OR⁴ where R⁴ = H or alkyl or aryl or functionalised alkyl or aryl groups.

3. Carbon-containing material with organic groups according to claim 1, **characterized in that** the donor substituents are alkyl or aryl groups, OR⁵ or N(R⁵)₂ where R⁵ = H or alkyl or aryl or functionalised alkyl or aryl groups.

4. Carbon-containing material with organic groups according to claim 1, **characterized in that** the carbon-containing material is carbon black.

5. Process for preparing a carbon-containing material according to claim 1, **characterized in that** the carbon-containing material is reacted with organic compounds of the general formula 1.

6. Use of the carbon-containing material according to claim 1 as filler, reinforcing filler, UV stabiliser, conductivity carbon black or pigment in rubber, plastics, printing inks, inks, inkjet inks, lacquers and colorants, bitumen, concrete and other constructional materials or paper.

7. Organic compound for preparing the carbon-containing material with organic groups according to claim 1, **characterized in that** this corresponds to the general formula 2 or 3, 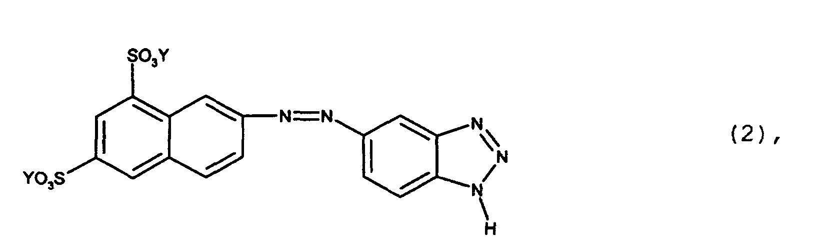 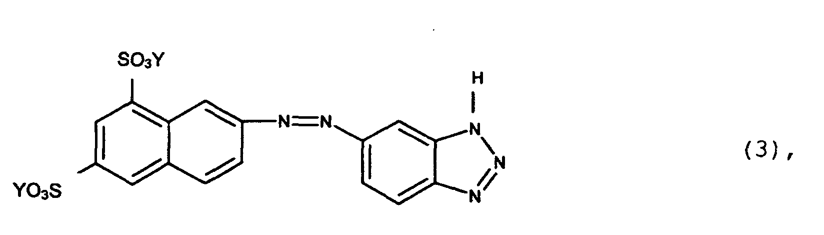 wherein Y represents H, metal^{u*} (u = 1-3) or N⁺R₄.

8. Dispersion, **characterized in that** it contains the carbon-containing material with organic groups according to claim 1.

9. Use of the dispersion according to claim 8 in printing inks, inks, lacquers and colorants.

## Revendications

1. Matériaux carbonés avec des groupes organiques,
**caractérisés en ce qu'**
ils sont obtenus en utilisant la réaction d'un matériau carboné avec des composés organiques de formule générale 1, 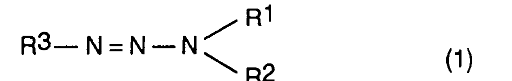 dans laquelle R¹, R², et R³ sont identiques ou différents et représentent H, un groupe alkyle ou aryle avec un substituant qui est accepteur ou donneur et/ou un groupe hydrophile ou un groupe hydrophobe , ou R¹ et R³ avec trois atomes d'azote forment un système cyclique et R² est H, un groupe alkyle ou aryle avec un groupe qui est accepteur ou un groupe hydrophobe.

2. Matériaux carbonés avec des groupes organiques selon la revendication 1,
**caractérisés en ce que**
les substituants accepteurs sont -COOR⁴, -CO-R⁴, -CN, -SO₂R⁴ ou -SO₂OR⁴ avec R⁴ = H, alkyle, aryle, alkyle fonctionnalisé ou aryle fonctionnalisé.

3. Matériaux carbonés avec des groupes organiques selon la revendication 1,
**caractérisés en ce que**
les substituants donneurs sont un groupe alkyle ou aryle, OR⁵ ou N(R⁵)₂ avec R⁵ = H, alkyle, aryle, alkyle fonctionnalisé ou aryle fonctionnalisé.

4. Matériaux carbonés avec des groupes organiques selon la revendication 1,
**caractérisés en ce que**
les matériaux carbonés sont des noirs de flamme.

5. Procédé de fabrication de matériaux carbonés avec des groupes organiques selon la revendication 1,
**caractérisé en ce que**
les matériaux carbonés entrent en réaction avec des composés organique de la formule générale 1.

6. Utilisation des matériaux carbonés avec des groupes organiques selon la revendication 1, comme matières de remplissage, agents de renforçateurs, stabilisateurs UV, conducteurs électriques ou pigments dans des caoutchoucs, dans des matières plastiques, dans des couleurs d'impression, dans des encres, dans des encres pour jet d'encre, dans des vernis, dans des toners et dans des peintures, dans des bitumes, dans des bétons et dans d'autres matériaux de construction ou dans du papier.

7. Composés organiques pour la fabrication les matériaux carbonés avec les groupes organiques selon la revendication 1,
**caractérisés en ce que**
ces composés organique sont de formules générales 2 ou 3 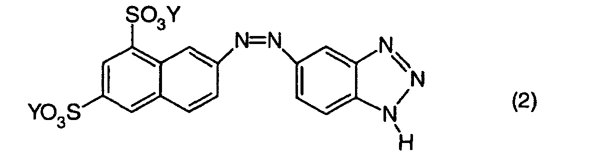 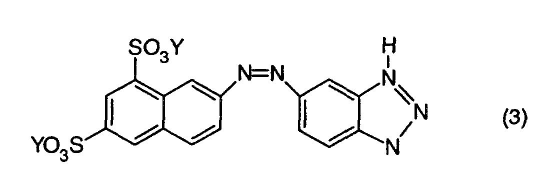 dans lesquelles Y est H, un métal^{u+} (u=1-3) ou N⁺R₄.

8. Dispersions
**caractérisées en ce qu'**
elles contiennent les matériaux carbonés avec des groupes organiques selon la revendication 1.

9. Utilisation des dispersions selon la revendication 8, dans des couleurs d'impression, des encres, des vernis, des toners et des peintures.
